(19) Europäisches Patentamt
European Patent Office
Office européen des brevets

(11) Veröffentlichungsnummer : **0 134 795**
**B1**

(12) # EUROPÄISCHE PATENTSCHRIFT

(45) Veröffentlichungstag der Patentschrift : **17.08.88**

(51) Int. Cl.⁴ : **C 07 D207/16**, C 07 D207/22, A 61 K 31/40

(21) Anmeldenummer : **84900529.3**

(22) Anmeldetag : **20.01.84**

(86) Internationale Anmeldenummer : **PCT/HU 84/00004**

(87) Internationale Veröffentlichungsnummer : **WO/8402906 (02.08.84 Gazette 84/18)**

(54) **NEUE 2- (N-(2,2,5,5-TETRAMETHYL-3-PYRROLIN-BZW.PYRROLIDIN-3-CARBONYL))-AMINO-DERIVATE.**

(30) Priorität : **20.01.83 HU 17883**

(43) Veröffentlichungstag der Anmeldung : **27.03.85 Patentblatt 85/13**

(45) Bekanntmachung des Hinweises auf die Patenterteilung : **17.08.88 Patentblatt 88/33**

(84) Benannte Vertragsstaaten : **AT BE CH DE FR GB LI NL**

(56) Entgegenhaltungen :
**CH-A- 336 068**
**GB-A- 1 057 290**
**GB-A- 1 160 527**
**US-A- 3 299 097**
**US-A- 3 624 081**

(73) Patentinhaber : **ALKALOIDA VEGYéSZETI GYáR**
**Postfach 1**
**H-4440 Tiszavasvári (HU)**

(72) Erfinder : **HIDEG, Kálmán**
**Ifjuság ut 5**
**H-7624 Pécs (HU)**
Erfinder : **HANKOVSZKY, Olga, H.**
**Ifjuság ut 5**
**H-7624 Pécs (HU)**
Erfinder : **FRANK, Lászlò**
**Elmunkás u. 23**
**H-4440 Tiszavasvári (HU)**
Erfinder : **BODI, Ilona**
**Elmunkás u. 27**
**H-4440 Tiszavasvári (HU)**
Erfinder : **CSAK, Jòzsef**
**Elmunkás u. 21**
**H-4440 Tiszavasvári (HU)**

(74) Vertreter : **Hansen, Bernd, Dr.rer.nat. et al**
**Hoffmann, Eitle & Partner Patentanwälte Arabellastrasse 4**
**D-8000 München 81 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 134 795 B1**

**Beschreibung**

Die Erfindung betrifft neue chemische Verbindungen, Verfahren zu ihrer Herstellung und pharmazeutische Mittel, welche diese Verbindungen enthalten.

Aus US-A-3 624 081 sind N-Acyl-2-ethylidenderivate von Pyrrolidin, die antibakterielle Eigenschaften haben, bekannt. In GB-A-1 057 290 werden N-Alkyl-5-dimethoxyphenyl-pyrrolidin-2-carboxamid-derivate mit pharmakologischen Eigenschaften beschrieben. Weiterhin sind aus CH-A-336 068 Amide von N-Alkylpiperidin-monocarbonsäuren oder N-Alkyl-pyrrolidin-monocarbonsäuren, die als Lokalanasthetika geeignet sind, bekannt.

Die Erfindung betrifft Verbindungen der allgemeinen Formel (I)

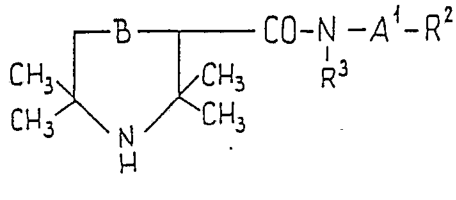

(I)

und ihre Salze, worin bedeuten :

$A^1$ einen Valenzstrich oder eine geradkettige oder verzweigte Alkylengruppe mit 1 bis 5 Kohlenstoffatomen bedeutet, welche mit einer Hydroxyl- oder Amino-Carbonyl-Gruppe substituiert sein kann,

B einen Valenzstrich oder eine Doppelbindung,

$R^2$ eine Phenyl-, Pyridyl-, Phenylamino- oder Phenyloxygruppe bedeutet, die mit ein oder mehrere 1 bis 4 Kohlenstoffatome enthaltenden Alkyl- oder Alkoxygruppen oder mit Halogenatomen substituiert sein können, oder eine Amino-Gruppe oder eine Diphenyl-methylgruppe oder eine $R^4$—NH—CO-Gruppe bedeutet, in welcher $R^4$ eine gegebenenfalls mit einer $C_1$-$C_4$-Alkyl- oder Alkoxygruppe oder mit Halogen substituierte Phenylgruppe bedeutet,

$R^3$ Wasserstoff oder eine 1 bis 4 Kohlenstoffe enthaltende Alkylgruppe.

Die erfindungsgemäßen Verbindungen der allgemeinen Formel (I) sind als Wirkstoff in pharmazeutischen Mitteln geeignet. Sie weisen insbesondere eine antiarhythmische Wirkung auf. Die Wirksamkeit kann mit bekannten, in der Therapie allgemein akzeptierten Mitteln wie Chinidin, Lidocain®, Procainamid verglichen werden und übertrifft häufig die Wirkung der bekannten Mittel.

Ein weiterer Vorteil der erfindungsgemäßen Verbindungen ist darin zu sehen, daß bei einer antiarhythmisch wirkenden Dosis keine Arhythmie der Herzkammer hervorgerufen wird, keine Bradycardie erfolgt und der Blutdruck bei den Versuchstieren nicht oder nur in geringem Maße vermindert wird.

Die erfindungsgemäßen Verbindungen der allgemeinen Formel (I) oder deren Salze können zur Prophylaxe und Therapie gegen Störungen des Herzrhythmus parenteral oder auch enteral verabreicht werden.

Die pharmakologische Wirkung der erfindungsgemäßen Verbindungen der allgemeinen Formel (I) wird nachfolgend bei einem Aconitin-Arrhythmie-Modell bei Ratten gezeigt (Zetler and Strubelt Arzneim.-Forsch. (Drug.-Res.) 30, 1497, 1980).

Wistar-Ratten (200-220 g) werden mit Urethan narkotisiert. Die Vena jugularis wird mit einer Polyethylenkanüle verbunden. Es wird eine Kontrolle-EKG-Aufnahme mit Standard-EGK-II-Ableitung vorgenommen. Die Testsubstanzen und die als Kontrolle verwendete physiologische Kochsalzlösung wird 5 Minuten vor der Aconitin-Infusion intravenös verabreicht. Die Aconitin-Lösung wird kontinuierlich in einer Dosis von 5 μg/kg mit einer Geschwindigkeit von 0,1 ml (100 g/Minute) als Infusion in die Vena jugularis eingeleitet. Dieser Vorgang wird bis zum Auftreten einer isoelektrischen EKG-Linie fortgesetzt.

Bei dem Versuch wurden die Zeitpunkte des Auftretens der ventricularen Extrasystole, ventrikulären Tachycardie und des Kammerflimmerns bestimmt. Einige Ergebnisse sind in der Tabelle II zusammengefaßt.

Auf Basis des verspäteten Eintretens der ventrikulären Extrasystole wurden $ED_{125}$ und $ED_{150}$-Werte bestimmt. Die akuten Toxizitäten wurden mit Mäusen (CFLP-Stamm) bestimmt und die $LD_{50}$-Werte wurden nach einer Woche nach der Lichtfield-Wilcoxon-Methode berechnet. Die antiarhythmisch wirkenden effektiven Dosen und die $LD_{50}$-Werte einiger Verbindungen sind in Tabelle 3 angegeben.

Die erfindungsgemäßen Verbindungen können in bekannter Weise zu Arzneimitteln verarbeitet werden, wobei man bekannte Methoden der pharmazeutischen Industrie anwendet. Hierzu gibt man Verdünnungsmittel, Träger und andere Hilfsmittel zu. Das Arzneimittel kann in Form von Tabletten, Dragees, Kapseln oder in ähnlichen peroralen Formen vorliegen. Die Mittel können auch parenteral, insbesondere i. v. Injektionen verabreicht werden. Das Gewebe wird von den Wirkstoffen nicht angegriffen.

Die Verbindungen der allgemeinen Formel I können auch vorteilhaft in Form von physiologisch neutralen Salzen angewendet werden. Hierzu werden vorteilhaft salzsaure, schwefelsaure oder sulfonsaure Salze oder Salze mit Bromwasserstoff verwendet.

Unter $C_1$-$C_4$-Alkyl- oder- Alkoxygruppen oder Halogenatome, die als Substituenten für die Gruppe

beim Rest $R^2$ in Frage kommen, sind beispielsweise Methyl, Ethyl, Isopropyl, Methoxy, Ethoxy, Chlor und Brom.

Die Verbindungen der allgemeinen Formel V sind Zwischen-produkte zur Herstellung der Verbindungen der allgemeinen Formel I.

Die Erfindung betrifft auch ein Verfahren zur Herstellung von Verbindungen der allgemeinen Formel I, das dadurch gekennzeichnet ist, daß man ein Piperidinonderivat der allgemeiner Formel (II)

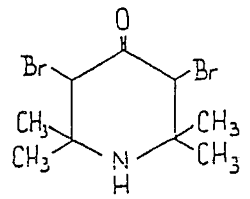

(II)

oder ein Salz davon mit einem Amin der allgemeinen Formel (III)

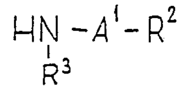

(III)

worin $A^1$, $R^2$ und $R^3$ die oben angegebene Bedeutung haben, umsetzt und gegebenenfalls das erhaltene Carboxamid der allgemeinen Formel (IV)

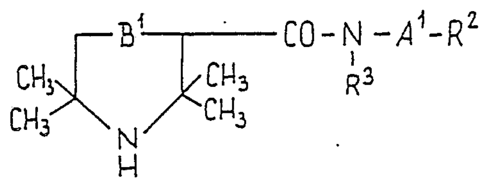

(IV)

worin

$A^1$, $R^2$ und $R^3$ die im Anspruch 1 angegebene Bedeutung haben, und

$B^1$ eine Einzel- oder eine Doppelbindung bedeutet, durch Reduktion im Pyrrolinring sättigt und gegebenenfalls durch Umsetzen mit einer Säure in ein Salz überführt.

Es ist aus der Literatur bekannt, dass man verschiedene aminoalkylsubstituierte Carboxamide aus dem Dibromderivat der Formel II herstellen kann (Golding, Ioannou, O'Brien ; Synthesis, 1975, 462).

Die Verbindungen der Formel II werden vorteilhaft den wässrigen Lösungen von Verbindungen der allgemeinen Formel III zugesetzt. Es ist zweckmässig, das antstandene Produkt durch Extraktion mit einem wasserunlöslichen Lösungsmittel zu isolieren.

Die Reaktion verläuft allgemein bei Raumtemperatur, es können jedoch auch höhere Temperaturen verwendet werden.

Als Ausgangsstoff kann man auch ein Salz von Piperidin, insbesondere das Bromwasserstoffsalz, vorteilhaft verwenden.

Als wasserunlösliches Lösungsmittel ist es zweckmässig, halogenierte Kohlenwasserstoffe wie Chloroform zu verwenden. Die Reaktion kann in Gegenwart einer organischen oder anorganischen Base durchgeführt werden.

Die Aufarbeitung des Reaktionsgemisches erfolgt zweckmässig wie folgt.

Die Lösung in einem wasserunlöslichem Lösungsmittel wird über Natriumsulphat oder Magnesiumsulphat getrocknet worauf das Lösungsmittel und etwaiges überschüssiges Diamin abgetrieben wird. Das Produkt wird aus dem Rest durch Kristallisation erhalten. Die Kristallisation kann durch Zusetzen eines Lösungsmittels wie Chloroform und/oder Ether beschleunigt werden.

Zur Herstellung von Verbindungen der allgemeinen Formel VII

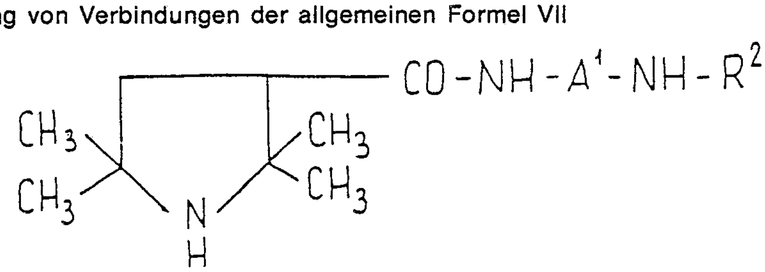

(VII)

werden die Pyrrolidin-Derivate einer reduktion unterworfen. Die Reduktion kann als katalytische Hydrierung, zweckmäßig in Gegenwart eines Palladium-Kohle-Katalysators, durchgeführt werden. Die Hydrierung erfolgt zweckmäßig bei Atmosphärendruck in Gegenwart eines organischen Lösungsmittels. Als Lösungsmittel kann man vorteilhaft wasserunlösliche organische Lösungsmittel, wie Chloroform, verwenden.

Die Aufarbeitung des Reaktionsgemisches erfolgt nach dem Entfernen des Katalysators durch Extraktion und Einengen des Extraktes.

Die Verbindungen der allgemeinen Formel I können auch als Zwischenprodukte für weitere Synthesen verwendet werden. In diesem Fall müssen die Produkte aus den Reaktionsgemischen häufig nicht isoliert werden.

Die Verbindungen der allgemeinen Formel I, in welcher B einen Valenzstrich, also eine Einzelbindung bedeutet, sind optisch aktiv und können rechts- oder linksdrehend sein. Sowohl die beiden optisch aktiven Formeln als auch das Racemat sind in die Erfindung eingeschlossen.

Die Erfindung wird in den Beispielen näher beschrieben.

Beispiel 1

4,31 g 2-Amino-3-(2,6-dimethyl-phenoxy)-propan Hydrochlorid werden in 60 ml Ethanol gelöst und mit 20 ml Wasser verdünnt. Nach Zusetzen von 8,3 g Kaliumcarbonat werden bei Raumtemperatur portionenweise 7,88 g 3,5-Dibrom-2,2,6,6-tetramethyl-4-piperidon-Hybrobromid zugegeben. Nach 3 Stunden wird die Lösung mit etwa 120 g Kaliumcarbonat gesättigt und 4-mal mit je 20 ml Chloroform extrahiert. Der Extrakt wird mit Wasser gewaschen, über Magnesiumsulfat getrocknet und filtriert. Das Filtrat wird bis zur Trockne eingedampft, worauf der Rückstand in 30 ml Ether gelöst und mit 40 ml n-Hexan verdünnt wird. Der ausgeschiedene Niederschlag wird kalt filtriert und getrocknet. Es werden 5,24 g (82 %) 2-[N-(2,2,5,5 Tetramethyl-2,5-dihydro-3-pyrrolin-3-carbonyl)]-3-(2,6-dimethyl-phenoxy)-aminopropan erhalten. (Siehe Verbindung Nr. 9 in Tabelle I).

Die Verbindungen Nr. 1-10 der Tabelle I, wurden aus den entsprechenden Aminen wie oben hergestellt. Die Daten sind in der Tabelle angeführt.

Beispiel 2

3,29 g des nach Beispiel 1, hergestellten Produktes werden in 20 ml Chloroform gelöst worauf in Gegenwart eines Pd/Kohle Katalysators von hoher Aktivität solange Wasserstoff eingeführt wird, bis keine Aufnahme mehr erfolgt. Die erhaltene Lösung wird filtriert und eingeengt und — wie in Beispiel 1, beschrieben wurde — aufgearbeitet. Das Produkt ist 2-[N-(2,2,5,5-Tetramethyl-3-pyrrolidin-3-carbonyl)]-3-(2,6-dimethyl-phenoxy)-amino-propan. (Verbindung Nr. 11 in Tabelle I).

Beispiele 3, 4 und 5

31,30 g 2,2,6,6-Tetramethyl-3,5-dibrom-piperid-4-on werden binnen einer Stunde zu folgenden Lösungen gegeben die jeweils auf 0 °C abgekühlt sind :

a) eine Lösung von 27,04 g 1,3-Diamino-2-propanol in 500 ml Wasser,

b) eine Lösung von 9,01 g 1,3-Diamino-2-propanol und 20,20 g Triethylamin in 500 ml Wasser, und

c) eine Lösung von 9,01 g 1,3-Diamino-2-propanol und 13,82 g Kaliumcarbonat in 500 ml Wasser.

Das Reaktionsgemisch wird eine weitere Stunde lang bei 0 °C umgerührt. Hierauf wird so viel Kaliumcarbonat (ungefähr 200 g) zugesetzt, bis das Produkt als obere Phase auf der Oberfläche der wässrigen Phase erscheint. Nach Extraktion mit Chloroform (3 × 100 ml) wird die Chloroformlösung über Magnesiumsulfat getrocknet. Das Lösungsmittel und der gegebenenfalls anwesende Überschuss an Amin wird bei vermindertem Druck abgetrieben. Der Rückstand wird in 150 ml Ether gelöst. Das ausgeschiedene N-(2-Hydroxy-3-amino-propyl)-2,2,5,5-tetramethyl-3-pyrrolin-3-carboxamid wird in kristalliner Form filtriert, gewaschen und getrocknet. (Verbindung Nr. 15 in Tabelle I).

Beispiele 6, 7 und 8

39,39 g des bromwasserstoffsauren Salzes von 2,2,6,6-Tetramethyl-3,5-dibrom-piperid-4-on werden zu folgenden Lösungen gegeben die jeweils auf 0 °C abgekühlt worden sind :

a) eine Lösung von 36,05 g 1,3-Diamino-2-propanol in 500 ml Wasser, oder

b) eine Lösung von 9,01 g 1,3-Diamino-2-propanol und 3,03 g Triethylamin in 500 ml Wasser, und

c) eine Lösung von 9,01 g 1,3-Diamino-2-propanol und 20,73 g Kaliumcarbonat in 500 ml Wasser.

Man verfährt wie in den Beispielen 3 bis 5. Das erhaltene Produkt entspricht dem Produkt der Beispiele 3 bis 5 (Verbindung Nr. 15 in Tabelle I).

Beispiel 9

22,53 g N-(3-Amino-propyl)-2,2,5,5-tetramethyl-3-pyrrolin-3-carboxamid (Verbindung Nr. 13 in Tabelle I) werden in 300 ml Chloroform gelöst und in Gegenwart eines Pd/Kohle Katalysators von hoher Aktivität solange mit Wasserstoff versetzt, bis keine Wasserstoffaufnahme mehr erfolgt. Das Reaktionsgemisch wird — wie in Beispiel 1 beschrieben wurde — aufgearbeitet. Das erhaltene ölartige Produkt kann unmittelbar für weitere Synthesen gebraucht werden. Das Produkt ist N-(3-Amino-propyl)-2,2,5,5-tetramethyl-pyrrolidin-3-carboxamid (Verbindung Nr. 18 in Tabelle I).

Tabelle I

$R^1-CON(R^3)-A^1-R^2$

| No | $R^1$ | $A^1$ ⋮ $R^2$ | $R^3$ | A.b % | Schmp. C° | Analyse /Mol. Gew./ | Analyse %ber/gef. C | H | N | Cl | $^1$H-NMR δ (ppm) |
|---|---|---|---|---|---|---|---|---|---|---|---|
| 1 | 2 | 3 | 4 | 5 | 6 | 7 | 8 | | | | 9 |
| 1 | 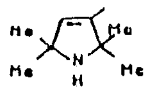 | 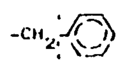 | H | 60 | 100-109 | $C_{16}H_{22}N_2O$ .HCl (294.82) | 65.18<br>65.17 | 7.86<br>8.12 | 9.50<br>9.58 | 12.02<br>12.22 | 1.50 (s,6H,2$CH_3$); 1.60 (s,6H,2$CH_3$); 4.30 (s,2H,$CH_2$); 6.22 (s,1H,CH=); 7.20 (s,5H,ArCH=) ($D_2O$). |
| 2 | 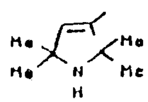 | 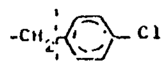 | H | 71 | 216-218 | $C_{16}H_{21}ClN_2O$.HCl (329.26) | 58.36<br>58.40 | 6.74<br>7.00 | 8.51<br>8.52 | 21.53<br>21.67 | 1.52 (s,6H,2$CH_3$); 1.62 (s,6H,2$CH_3$); 4.26 (s,2H,$CH_2$); 6.26 (s,1H,CH=); 7.14 (s,4H,ArCH=) ($D_2O$). |
| 3 | 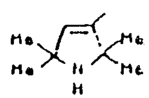 | 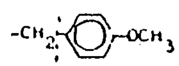 | H | 69 | 82-83 | $C_{17}H_{24}N_2O_2$ (288.39) | 70.80<br>70.99 | 8.39<br>8.34 | 9.71<br>9.46 | -<br>- | 1.24 (s,6H,2$CH_3$); 1.44 (s,6H,2$CH_3$); 3.65 (s,3H,$OCH_3$); 4.37 (s,2H,$CH_2$); 6.02 (s,1H,CH=); 6.70-7.30 (m,4H,ArCH=).($D_2O$) |
| | | | | | 214-216 | $C_{17}H_{24}N_2O_2$.HCl (324.85) | 62.66<br>62.62 | 7.76<br>7.86 | 8.62<br>8.56 | 10.91<br>10.75 | 1.52 (s,6H,2$CH_3$); 1.62 (s,6H,2$CH_3$); 3.66 (s,3H,$OCH_3$); 4.24 (s,2H,$CH_2$); 6.22 (s,1H,CH=); 6.70-7.80 (m,4H,ArCH=) ($D_2O$). |
| 4 | 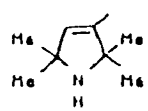 | 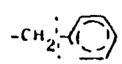 | $CH_3$- | 50 | 225-226 | $C_{17}H_{24}N_2O$.HCl (308.87) | 66.11<br>66.16 | 8.16<br>8.27 | 9.07<br>9.06 | 11.48<br>11.52 | 1.52 (s,6H,2$CH_3$); 1.60 (s,6H,2$CH_3$); 2.90 (s,3H,$NCH_3$); 4.58 (s,2H,$CH_2$); 6.04 (s,1H,CH=); 7.10-7.60 (m,5H, ArCH=) (DMSO). |
| 5 | 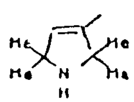 | 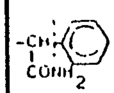 | H | 93 | 220-221 | $C_{17}H_{23}N_3O_2$ (301.39) | 67.75<br>67.59 | 7.69<br>7.71 | 13.94<br>13.61 | - | 1.30 (s,6H,2$CH_3$); 1.40 (s,6H,2$CH_3$); 5.62 (s,1H,CH); 6.22 (s,1H,CH=); 7.30-7.45 (m,5H,ArCH=) ($CDCl_3$). |
| | | | | | 220-223 | $C_{17}H_{23}N_3O_2$.HCl (337.87) | 60.44<br>60.43 | 7.16<br>7.25 | 12.44<br>12.43 | 10.49<br>10.43 | 1.52 (s),1.54 (s), 1.60 (s), 1.66 (s), (12H,4$CH_3$); 5.40 (s,1H,CH); 6.40 (s,1H, CH=); 7.36 (s,5H,ArCH=),($D_2O$) |

A.b : Ausbeute

Tabelle I (Fortsetzung)

| 1 | 2 | 3 | 4 | 5 | 6 | 7 | 8 | | | | 9 |
|---|---|---|---|---|---|---|---|---|---|---|---|
| 6 | 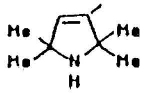 | 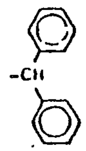 | H | 97 | 170-171 | $C_{22}H_{26}N_2O$<br>(334.46) | 79.00<br>79.20 | 7.84<br>7.67 | 8.38<br>8.51 | – | 1.26 (s,6H,$2CH_3$); 1.42 (s,6H,$2CH_3$); 6.08 (s,1H,CH); 6.24 (s,1H,CH); 7.26 (s,10H,ArCH=) ($CDCl_3$). |
| | | | | | 245-247 | $C_{22}H_{26}N_2O.HCl$<br>(370.92) | 71.24<br>70.98 | 7.34<br>7.31 | 7.55<br>7.35 | 9.56<br>9.86 | 1.52 (s,6H,$2CH_3$); 1.60 (s,6H,$2CH_3$); 6.10 (s,1H,CH); 6.30 (s,1H,CH); 7.29 (s,10H,ArCH=) ($C_2O$). |
| 7 | 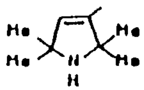 | 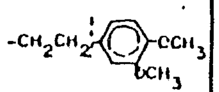 | H | 45 | 212-214 | $C_{19}H_{28}N_2O_3.HCl$<br>(368.92) | 61.86<br>61.76 | 7.92<br>8.03 | 7.59<br>7.61 | 9.61<br>9.86 | 1.52 (s,12H,$4CH_3$); 2.50-2.85 (m,2H,$CH_2$); 3.25-3.65 (m,2H,$CH_2$); 3.76 (s,6H,$2OCH_3$); 6.05 (s,1H,CH=); 6.70-7.0 (m,3H,ArCH=) ($D_2O$). |
| 8 | 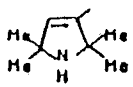 | 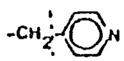 | H | 68 | 198-200 | $C_{15}H_{21}N_3O.HCl$<br>(332.29) | 54.22<br>54.37 | 6.98<br>7.09 | 12.65<br>12.49 | 21.34<br>21.48 | 1.60 (s,6H,$2CH_3$); 1.66 (s,6H,$2CH_3$); 4.70 (s,2H,$CH_2$); 6.58 (s,1H,CH=); 7.80-8.05 (m,2H,PyCH=), 8.60-8.80 (m, 2H,PyCH=) ($D_2O$). |
| 9 | 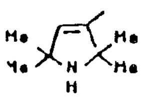 | 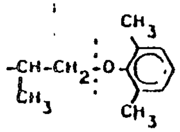 | H | 82 | 134-135 | $C_{20}H_{30}N_2O_2$<br>(330.47) | 72.69<br>72.67 | 9.15<br>8.92 | 8.48<br>8.44 | – | 1.28 (s,$2CH_3$), 1.45 (s,$2CH_3$), 1.43 (d, $CH_3$) (15H); 2.24 (s,6H,$2CH_3$); 3.50-4.0 (m,2H,$CH_2$); 4.0-4.50 (m,1H,CH); 6.08 (s,1H,CH=); 6.84 (s,3H,ArCH=) ($CDCl_3$). |
| | | | | | 218 (bcmlik) | $C_{20}H_{30}N_2O_2.HCl$<br>(366.93) | 65.47<br>65.41 | 8.52<br>8.32 | 7.63<br>7.60 | 9.66<br>9.82 | 1.24 (d,3H,$CH_3$); 1.58 (s,6H,$2CH_3$); 1.68 (s,6H,$2CH_3$); 2.14 (s,6H,$2CH_3$); 3.55-3.80 (m,2H,$CH_2$); 3.95-4.30 (m,1H, CH); 6.24 (s,1H,CH=); 6.90 (s,3H,ArCH=) ($D_2O$). |
| 10 | 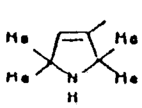 | 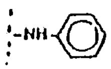 | H | 52 | 238-239 | $C_{15}H_{21}N_3O.HCl$<br>(295.82) | 60.91<br>61.06 | 7.50<br>7.34 | 14.20<br>14.21 | 11.98<br>12.05 | 1.68 (s,6H,$2CH_3$); 1.66 (s,6H,$2CH_3$); 6.46 (s,1H,CH=); 6.70-7.50 (m,5H, ArCH=) ($D_2O$). |
| 11 | 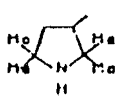 | 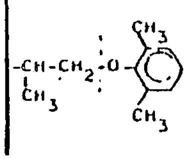 | H | 50 | 216-218 | $C_{20}H_{32}N_2O_2.HCl$<br>(368.94) | 65.11<br>64.98 | 9.02<br>9.15 | 7.59<br>7.65 | 9.61<br>9.83 | 1.0-1.80 (m, 15H, $4CH_3$, $CH_2CH$); 1.80-2.40 (m,9H,$3CH_3$); 2.70-3.80 (m,3H,$CHCH_2$) 6.66 (s,3H,ArCH=) ($C_2O$). |

Tabelle I (Fortsetzung)

| 1 | 2 | 3 | 4 | 5 | 6 | 7 | 8 | | | | 9 |
|---|---|---|---|---|---|---|---|---|---|---|---|
| 12. | 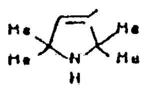 | $-CH_2CH_2$-$NH_2$ | H | 70 | 115-117 | $C_{11}H_{21}N_3O$ (211.32) | 62.53<br>62.64 | 10.02<br>10.14 | 19.89<br>19.88 | - | 1.26 (s,6H,2$CH_3$); 1.34 (s,6H;2$CH_3$); 2.70-3.05 (m,2H,$CH_2$); 3.20-3.65 (m,2H,$CH_2$); 6.10 (s,1H,CH=) ($CDCl_3$). |
| 13. | 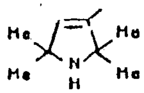 | $-CH_2CH_2CH_2$-$NH_2$ | H | 70 | 87-88 | $C_{12}H_{23}N_3O$ (225.35) | 63.97<br>64.06 | 10.29<br>10.33 | 18.65<br>18.72 | - | 1.28 (s,6H,2$CH_3$); 1.42 (s,6H,2$CH_3$); 1.50-1.95 (m,2H,$CH_2$); 2.70-3.20 (m,2H,$CH_2$); 3.24-3.65 (m,2H,$CH_2$); 6.10 (s,1H,CH=) ($CDCl_3$). |
| 14 | 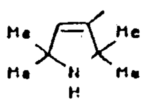 | $-CH_2$-CH-$NH_2$<br>$CH_3$ | H | 50 | 93-95 | $C_{12}H_{23}N_3O$ (225.35) | 63.96<br>63.89 | 10.29<br>10.32 | 18.65<br>18.69 | - | 1.12 (d,3H,$CH_3$); 1.30 (s,6H,2$CH_3$); 1.45 (s,6H,2$CH_3$); 2.70-3.55 (m,3H,$CH_2$CH); 6.10 (s,1H,CH=) ($CDCl_3$). |
| 15 | 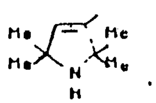 | $-CH_2$CH-$CH_2$-$NH_2$<br>OH | H | 68 | 83-85 | $C_{12}H_{23}N_3O_2$ (241.35) | 59.72<br>59.68 | 9.61<br>9.69 | 17.41<br>17.44 | - | 1.28 (s,6H,2$CH_3$); 1.42 (s,6H,2$CH_3$); 2.50-2.85 (m,2H,$CH_2$); 3.20-3.94 (m,3H,$CH_2$CH); 6.10 (s,1H,CH=) ($CDCl_3$); |
| 16 | 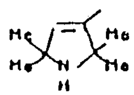 | $-CH_2CH_2CH_2CH_2$$NH_2$ | H | 55 | 108-111 | $C_{13}H_{25}N_3O$ (239.38) | 65.23<br>65.32 | 10.53<br>10.60 | 17.55<br>17.57 | - | 1.28 (s,6H,2$CH_3$); 1.42 (s,6H,2$CH_3$); 1.24-2.05 (m,4H,2$CH_2$); 3.10-3.55 (m,4H,2$CH_2$); 6.14 (s,1H,CH=) ($CDCl_3$). |
| 17 | 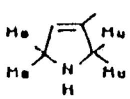 | $CH_3$<br>$-CH_2$-C-$NH_2$<br>$CH_3$ | H | 60 | 108-110 | $C_{13}H_{25}N_3O$ (239.38) | 65.23<br>65.30 | 10.53<br>10.61 | 17.55<br>17.51 | - | 1.18 (s,6H,2$CH_3$); 1.32 (s,6H,2$CH_3$); 1.50 (s,6H,2$CH_3$); 3.16 (d,2H,$CH_2$); 6.14 (s,1H,CH=) ($CDCl_3$). |
| 18 | 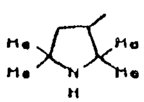 | $-CH_2CH_2CH_2$-$NH_2$ | H | 76 | olaj | $C_{12}H_{25}N_3O$ (227.36) | 63.40<br>63.58 | 11.08<br>11.19 | 18.48<br>18.36 | - | 0.95-1.45 (m,12H,4$CH_3$); 1.45-2.20 (m,5H,$CH_2$CH,$CH_2$); 2.52-3.05 (m,2H,$CH_2$); 3.10-3.45 (m,2H,$CH_2$) ($CDCl_3$). |

Tabelle I (Fortsetzung)

| 1 | 2 | 3 | 4 | 5 | 6 | 7 | 8 | 9 |
|---|---|---|---|---|---|---|---|---|
| 19. | 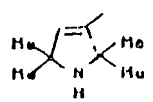 | 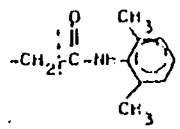 | H | 63 | 211-213 | $C_{19}H_{27}N_3O_2 \cdot$ HCl (365.90) | 63.37 7.71 11.48 9.69<br>63.18 7.91 11.49 9.72 | 1.57 (s,6H,2$CH_3$); 1.66 (s,6H,2$CH_3$); 2.10 (s,6H,2$CH_3$); 4.10 (s,2H,$CH_2$); 6.45 (s,1H,CH=); 7.10 (s,3H,ArCH=) ($D_2O$) |
| 20 | 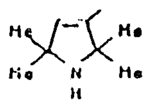 | 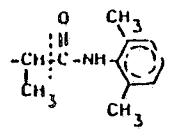 | H | 59 | 187-188 | $C_{20}H_{29}N_3O_2$ (343.47) | 69.94 8.51 12.23 -<br>69.90 8.29 12.29 | 1.26 (s,6H,2$CH_3$); 1.31 (s,6H,2$CH_3$); 1.52 (d,3H,$CH_3$,J=7.2 Hz); 2.15 (s, 6H,2$CH_3$); 4.86 (q,1H,CH,J=7.2 Hz); 6.20 (s,1H,CH=); 7.0 (s,3H,ArCH=) ($CDCl_3$) |
| | | | | | 168-169 | $C_{20}H_{29}N_3O_2 \cdot$ HCl (379.93) | 63.23 7.96 11.06 9.33<br>63.37 7.65 10.92 9.34 | 1.52 (d), 1.54 (s), 1.62 (s) (15H, 5$CH_3$); 2.10 (s,6H,2$CH_3$); 4.40-4.80 (m,1H,$D_2O$ alatt); 6.44 (s,1H,CH=); 7.10 (s,3H,ArCH=) ($D_2O$) |
| 21. | 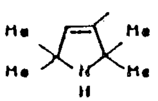 | 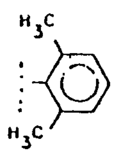 | H | 50 | 127-129 | $C_{17}H_{24}N_2O$ (272.41) | 74.96 8.88 10.28 -<br>75.06 8.97 10.35 | 1.38 (s,6H,2$CH_3$); 1.52 (s,6H,2$CH_3$); 2.24 (s,6H,2$CH_3$); 6.22 (s,1H,CH=); 7.08 (s,3H,ArCH=) ($CDCl_3$) |
| | | | | | 195-199 | $C_{17}H_{24}N_2O$ .HCl (308.87) | 66.11. 8.16 9.07 11.48<br>66.23 8.36 9.22 11.63 | 1.75 (s,6H,2$CH_3$); 1.85 (s,6H,2$CH_3$); 2.28 (s,6H,2$CH_3$); 6.66 (s,1H,CH=); 7.24 (s,3H,ArCH=) ($D_2O$) |

Tabelle II

Veränderungen der Erscheinungszeiten von EKG-Äenderungen (bei Ratten), welche durch eine Aconitin-Infusion hervorgerufen wurden /min ; X ± S.E.M./ n = Zahl der Versuchstiere

| Verbindung Nr. | Dosis mg/kg i.v. | n | Kammer-Extrasystole min ± S.E.M. | Kammer-Tachykardie min. ± S.E.M. | Fibrillo-Flattern min. ± S.E.M. |
|---|---|---|---|---|---|
| 0,9%-ige NaCl Lösung | | 15 | 6,1 ± 0,27 | 8,3 ± 0,42 | 11,7 ± 0,45 |
| 9. | 0,5 | 3 | 7,2 ± 0,06 | 11,1 ± 0,68 | 14,6 ± 0,48 |
| | 1,0 | 5 | 9,2 ± 0,10 | 15,3 ± 0,29 | 20,9 ± 0,50 |
| | 2,0 | 5 | 13,2 ± 1,21 | 18,9 ± 0,13 | 24,6 ± 0,79 |
| 11. | 0,25 | 4 | 7,6 ± 0,24 | 11,3 ± 0,88 | 15,9 ± 0,49 |
| | 0,5 | 5 | 9,9 ± 0,40 | 15,3 ± 0,52 | 19,1 ± 0,89 |
| | 1,0 | 5 | 13,2 ± 1,29 | 19,3 ± 1,24 | 23,5 ± 0,42 |
| 19. | 1,0 | 6 | 6,6 ± 0,32 | 10,4 ± 2,58 | 15,1 ± 0,70 |
| | 4,0 | 4 | 9,8 ± 0,34 | 16,1 ± 2,62 | 17,4 ± 1,22 |
| | 8,0 | 4 | 13,8 ± 0,35 | 27,0 ± 1,10 | 30,0 ± 1,72 |
| 20. | 1,0 | 4 | 7,1 ± 0,49 | 12,3 ± 0,42 | 15,5 ± 0,59 |
| | 2,0 | 4 | 9,2 ± 0,65 | 15,1 ± 0,22 | 19,5 ± 0,54 |
| | 4,0 | 6 | 11,7 ± 0,50 | 18,4 ± 0,75 | 23,5 ± 1,63 |
| Chinidin | 5,0 | 3 | 6,9 ± 0,06 | 10,4 ± 0,45 | 13,7 ± 0,44 |
| | 10,0 | 5 | 8,4 ± 0,42 | 11,9 ± 0,64 | 15,8 ± 1,00 |
| | 20,0 | 4 | 10,7 ± 0,58 | 15,7 ± 0,48 | 18,1 ± 0,22 |

Tabelle III

Effektive Dosen auf Aconitin Arrhythmie bei Ratten ; akute Toxizitätswerte (Konfidenzgrenzen 95 %). Bei Kontrolltieren, welche mit einer physiologischen Salzlösung vorbehandelt wurden, verursacht die Aconitin-Infusion von 5 µg/kg nach 6,1 Minuten eine Kammer-Extrasystole. Die $ED_{125}$ und $ED_{150}$ Werte entsprechen einer Infusionszeit von 7,6 und 9,2.

| Verbindung Nr. | $ED_{125}$ mg/kg i.v. | $ED_{150}$ mg/kg i.v. | $LD_{50}$ mg/kg i.v. |
|---|---|---|---|
| 9. | 0,6 /0,5-0,7/ | 0,9 /0,8-1,0/ | 11 /9-13/ |
| 11. | 0,26 /0,23-0,31/ | 0,39 /0,33-0,45/ | 1,96 /1,53-2,51/ |
| 19. | 1,53 /1,28-1,82/ | 2,44 /2,04-2,91/ | 53 /46-62/ |
| 20. | 1,21 /1,01-1,44/ | 1,91 /1,61-2,28/ | 15 /13-28/ |

Tabelle III (Fortsetzung)

| Verbindung Nr. | $ED_{125}$ mg/kg i.v. | $ED_{150}$ mg/kg i.v. | $LD_{50}$ mg/kg i.v. |
|---|---|---|---|
| Chinidin | 6,75 /5,4-8,3/ | 11,9 /9,6-14,8/ | - |
| 6. | 5,4 /3,1-9,4/ | 23,1 / 13-39,9/ | 63 / 56-70/ |

**Patentansprüche**

1. Verbindungen der allgemeinen Formel (I)

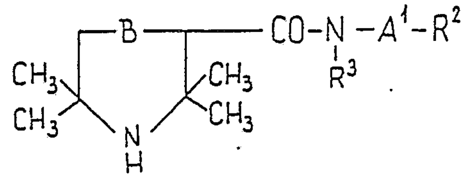

(I)

und ihre Salze, worin bedeuten :

$A^1$ eine Valenzstrich oder eine geradekettige oder verzweigte Alkylengruppe mit 1 bis 5 Kohlenstoffatomen bedeutet, welche mit einer Hydroxyl- oder Amino-Carbonyl-Gruppe substituiert sein kann,

B einen Valenzstrich oder eine Doppelbindung,

$R^2$ eine Phenyl-, Pyridyl-, Phenylamino- oder Phenyloxygruppe bedeutet, die mit ein oder mehrere 1 bis 4 Kohlenstoffatome enthaltenden Alkyl- oder Alkoxygruppen oder mit Halogenatomen substituiert sein können, oder eine Amino-Gruppe oder eine Diphenyl-methylgruppe oder eine $R^4$—NH—CO-Gruppe bedeutet, in welcher $R^4$ eine gegebenenfalls mit einer $C_{1-4}$-Alkyl- oder Alkoxygruppe oder mit Halogen substituierte Phenylgruppe bedeutet,

$R^3$ Wasserstoff oder eine 1 bis 4 Kohlenstoffe enthaltende Alkylgruppe.

2. Verbindungen gemäß Anspruch 1 der allgemeinen Formel (V)

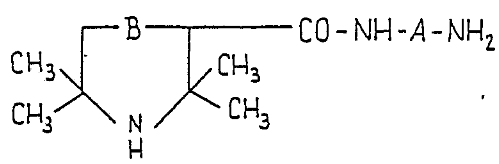

(V)

worin bedeuten :

A einen Valenzstrich oder eine gerade oder verzweigte Alkylengruppe mit 1 bis 4 Kohlenstoffatomen, welche mit einer Hydroxylgruppe substituiert sein kann, und

B eine Einzel- oder eine Doppelbindung und Salze davon mit Säuren.

3. Als Verbindung gemäß Anspruch 1, 2-(2,2,5,5-Tetramethyl-3-pyrrolin-3-carbonylamino)-N-(2,6-dimethyl-phenyl)-acetamid und Salze davon mit Säuren.

4. Als Verbindung gemäß Anspruch 1, N-(2,6-Dimethyl-phenyl)-2,2,5,5-tetramethyl-3-pyrrolin-3-carboxamid und Salze davon mit Säuren.

5. Als Verbindung gemäß Anspruch 1, 2-(2,2,5,5-Tetramethyl-3-pyrrolin-3-carbonylamino)-N-(2,6-dimethyl-phenyl)-propionamid und Salze davon mit Säuren.

6. Verfahren zur Herstellung von Verbindungen der allgemeinen Formel I gemäß Anspruch 1, dadurch gekennzeichnet, daß man ein Piperidinon-derivat der allgemeinen Formel (II)

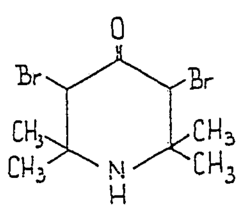

(II)

oder ein Salz davon mit einem Amin der allgemeinen Formel (III)

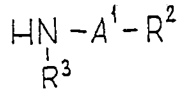

(III)

worin $A^1$, $R^2$ und $R^3$ die in Anspruch 1 genannte Bedeutung haben, umsetzt und gegebenenfalls das erhaltene Carboxamid der allgemeinen Formel (IV)

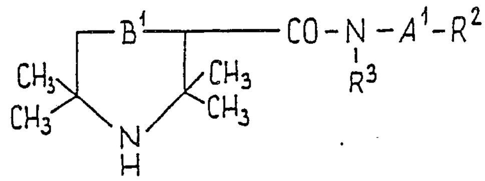

(IV)

worin

$A^1$, $R^2$ und $R^3$ die im Anspruch 1 angegebene Bedeutung haben, und

$B^1$ eine Einzel- oder eine Doppelbindung bedeutet, durch Reduktion im Pyrrolinring sättigt und gegebenenfalls durch Umsetzen mit einer Säure in ein Salz überführt.

7. Verfahren nach Anspruch 6, dadurch gekennzeichnet, daß die Reaktion in Anwesenheit eines säurebindenden Mittels durchgeführt wird.

8. Verfahren gemäß Anspruch 7, dadurch gekennzeichnet, daß man als säurebindendes Mittel ein Alkalicarbonat oder Alkalihydrocarbonat verwendet.

9. Verfahren nach Ansprüchen 6 bis 8, dadurch gekennzeichnet, daß die Verbindung der allgemeinen Formel (III) in Form des Hydrogenbromid-Salzes umgesetzt wird.

10. Verfahren nach Ansprüchen 6 bis 9, dadurch gekennzeichnet, daß der Pyrrolinring in Anwesenheit von Palladium durch Einführen von Wasserstoff gesättigt wird.

11. Verfahren zur Herstellung von Verbindungen der allgemeinen Formel (V) gemäß Anspruch 2, dadurch gekennzeichnet, daß man die Verbindung der Formel (II) in Form der Base oder eines Salzes mit einem Diaminoalkan der allgemeinen Formel (VI)

$$H_2N—A—NH_2 \qquad (VI)$$

in welcher A die in Anspruch 2 genannte Bedeutung hat, umsetzt und das erhaltene Produkt gegebenenfalls reduziert.

12. Pharmazeutisches Mittel, dadurch gekennzeichnet, daß es als Wirkstoff eine Verbindung der allgemeinen Formel (I) gemäß Anspruch 1 oder eines pharmazeutisch akzeptablen Salzes davon enthält.

13. Pharmazeutisches Mittel gemäß Anspruch 12 mit antiarhythmischer Wirkung.

14. Verwendung einer Verbindung der allgemeinen Formel (I) gemäß Anspruch 1 zur Herstellung eines Arzneimittels mit antiarhythmischer Wirkung.

**Claims**

1. Compounds of general formula I

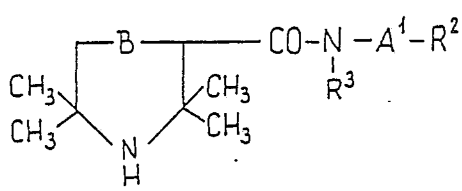

(I)

and their salts, wherein the following meanings apply :

$A^1$ a valency bond or a straight or branched chain alkylene group with 1 to 5 carbon atoms, which can be substituted with a hydroxyl or aminocarbonyl group,

B a valency bond or a double bond,

$R^2$ a phenyl, pyridyl, phenylamino or phenyloxy group, which can be substituted with one or more alkyl or alkoxy groups containing 1 to 4 carbon atoms or with halogen atoms, or an amino group or a diphenyl methyl group or a $R^4$—NH—CO group, in which $R^4$ is a phenyl group optionally substituted with a $C_{1-4}$-alkyl or alkoxy group or with halogen,

$R^3$ hydrogen or an alkyl group containing 1 to 4 carbon atoms.

2. Compounds according to claim 1 of general formula V

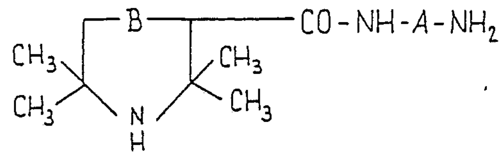 (V)

wherein the following meanings apply :

A a valency bond or a straight or branched chain alkylene group with 1 to 4 carbon atoms, which can be substituted with a hydroxyl group and

B a single or double bond and salts thereof with acids.

3. As a compound according to claim 1, 2-(2,2,5,5-tetramethyl-3-pyrroline-3-carbonylamino)-N-(2,6-dimethyl-phenyl)-acetamide and salts thereof with acids.

4. As a compound according to claim 1, N-(2,6-dimethyl-phenyl)-2,2,5,5-tetramethyl-3-pyrroline-3-carboxamide and salts thereof with acids.

5. As a compound according to claim 1, 2-(2,2,5,5-tetramethyl-3-pyrroline-3-carbonylamino)-N-(2,6-dimethyl-phenyl)-propionamide and salts thereof with acids.

6. Process for producing compounds of general formula I, which is characterized in that a piperidinone derivative of general formula II

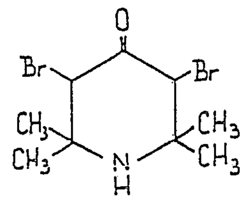 (II)

or a salt thereof is reacted with an amine of general formula III

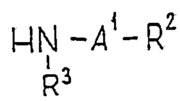 (III)

in which $A^1$, $R^2$ and $R^3$ have the meanings given hereinbefore and optionally the carboxamide obtained of general formula IV

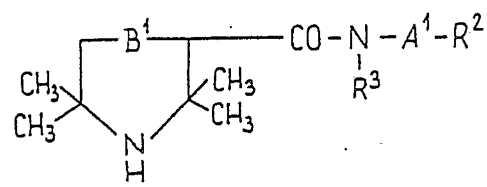 (IV)

in which

$A^1$, $R^2$ and $R^3$ have the meanings given in claim 1 and

$B^1$ is a single or a double bond is saturated by reduction in the pyrroline ring and is optionally converted into a salt by reacting with an acid.

7. Process according to claim 6, characterized in that the reaction is performed in the presence of an acid-binding agent.

8. Process according to claim 7, characterized in that an alkali metal carbonate or alkali metal hydrocarbonate is used as the acid-binding agent.

9. Process according to claims 6 to 8, characterized in that the compound of general formula III is reacted in the form of the hydrogen bromide salt.

10. Process according to claims 6 to 9, characterized in that the pyrroline ring is saturated by the introduction of hydrogen in the presence of palladium.

11. Process for the preparation of compounds of general formula V according to claim 2, characterized in that the compound of formula III in the form of the base or a salt is reacted with a diaminoalkane of general formula VI

$$H_2N—A—NH_2 \qquad (VI)$$

in which A has the meaning given in claim 2 and the product obtained is optionally reduced.

12. Pharmaceutical product, characterized in that as the active substance it contains a compound of general formula I according to claim 1 or a pharmaceutically acceptable salt thereof.

13. Pharmaceutical product according to claim 12 having an antiarrhythmic action.

14. Use of a compound of general formula I according to claim 1 for producing a medicament with an antiarrhythmic action.

**Revendications**

1. Composés de formule générale (I)

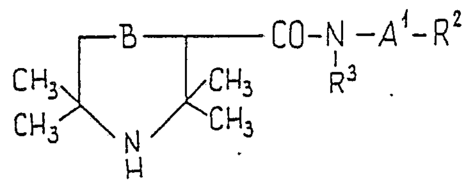

(I)

et leurs sels, où :

$A^1$ représente un trait de valence ou un groupe alcoylène à chaîne droite ou ramifiée en $C_1$ à $C_5$ qui peut être substitué par un groupe hydroxyle — ou amino-carbonyle,

B représente un trait de valence ou une double liaison,

$R^2$ représente un groupe phényle, pyridyle, phénylamino ou phényloxy qui peut être substitué par un ou plusieurs groupes alcoyles ou alcoxy comportant de 1 à 4 atomes de carbone, ou par des atomes d'halogènes, ou un groupe amino ou un groupe diphénylméthyle ou un groupe $R_4$—NH—CO—, où $R^4$ représente un groupe phényle éventuellement substitué par un groupe alcoyle ou alcoxy en $C_1$ à $C_4$ ou par un halogène,

$R^3$ représente un hydrogène ou un groupe alcoyle comportant de 1 à 4 atomes de carbone.

2. Composés selon la revendication 1 de formule générale (V)

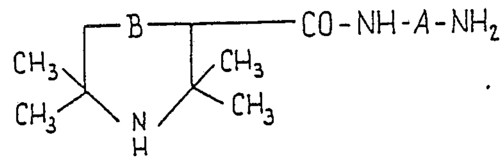

(V)

où :

A représente un trait de valence ou un groupe alcoylène à chaîne droite ou ramifiée comportant de 1 à 4 atomes de carbone, qui peut être substitué par un groupe hydroxyle, et

B représente une liaison simple ou double, et leurs sels avec des acides.

3. Comme composés selon la revendication 1, le 2-(2,2,5,5-tétraméthyl-3-pyrrolin-3-carbonylamino)-N-(2,6-diméthyl-phényl)-acétamide et ses sels avec des acides.

4. Composé selon la revendication 1, le N-(2,6-diméthyl-phényl)-2,2,5,5-tétraméthyl-3-pyrrolin-3-carboxamide et ses sels avec des acides.

5. Composé selon la revendication 1, le 2-(2,2,5,5-tétraméthyl-3-pyrrolin-3-carbonylamino)-N-(2,6-diméthyl-phényl)-propionamide et ses sels avec des acides.

6. Procédé de préparation de composés de formule générale (I) selon la revendication 1, caractérisé en ce qu'on fait réagir un dérivé de pipéridinone de formule générale (II)

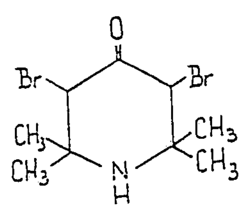

(II)

ou un de ses sels avec une amine de formule générale (III)

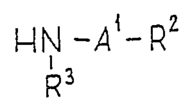

(III)

où $A^1$, $R^2$ et $R^3$ ont la signification donnée dans la revendication 1, et éventuellement en ce qu'on sature le carboxamide de formule générale (IV) obtenu

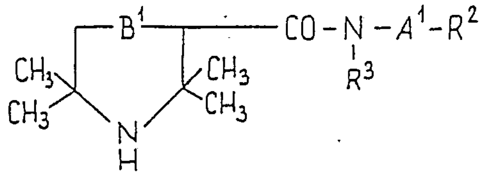

(IV)

où

$A^1$, $R^2$ et $R^3$ ont la signification donnée dans la revendication 1, et

$B^1$ représente une liaison simple ou double, par réduction dans le noyau pyrrolidine, et éventuellement en ce qu'on le transforme par réaction avec un acide en un sel.

7. Procédé selon la revendication 6, caractérisé en ce qu'on conduit la réaction en présence d'un agent liant d'acide.

8. Procédé selon la revendication 7, caractérisé en ce qu'on utilise comme agent liant d'acide un carbonate alcalin ou un hydrocarbonate alcalin.

9. Procédé selon les revendications 6 à 8, caractérisé en ce qu'on fait réagir le composé de formule générale (III) sous la forme du sel de bromhydrate.

10. Procédé selon les revendications 6 à 9, caractérisé en ce qu'on sature le noyau pyrrolidine en présence de palladium par introduction d'hydrogène.

11. Procédé de préparation de composés de formule générale (V) selon la revendication 2, caractérisé en ce qu'on fait réagir le composé de formule (II) sous la forme de la base ou d'un sel avec un diaminoalcane de formule générale (VI)

$$H_2N—A—NH_2 \qquad (VI)$$

où A a la signification donnée dans la revendication 2 et en ce qu'on réduit éventuellement le produit obtenu.

12. Agent pharmaceutique caractérisé en ce qu'il contient comme substance active un composé de formule générale (I) selon la revendication 1 ou un de ses sels pharmaceutiquement acceptables.

13. Agent pharmaceutique selon la revendication 12 à action anti-arythmique.

14. Application d'un composé de formule générale (I) selon la revendication 1 à la préparation d'un médicament à action anti-arythmique.